**Europäisches Patentamt**

**European Patent Office**

**Office européen des brevets**

(19)

(11) Numéro de publication : **0 433 140 B1**

## (12) FASCICULE DE BREVET EUROPEEN

(45) Date de publication du fascicule du brevet :
**31.03.93 Bulletin 93/13**

(51) Int. Cl.$^5$ : **A61K 9/06,** A61K 47/38, A61K 37/02

(21) Numéro de dépôt : **90403466.7**

(22) Date de dépôt : **06.12.90**

(54) **Nouvelle préparation pharmaceutique anti-allergique à action prolongée.**

(30) Priorité : **11.12.89 FR 8916339**

(43) Date de publication de la demande :
**19.06.91 Bulletin 91/25**

(45) Mention de la délivrance du brevet :
**31.03.93 Bulletin 93/13**

(84) Etats contractants désignés :
**AT BE CH DE DK ES GB GR IT LI LU NL SE**

(56) Documents cités :
**FR-A- 2 546 407**

(73) Titulaire : **THEA (Thérapeutique et Applications) S.A.
12, rue Louis Blériot, B.P. 88 Saint-Jean
F-63016 Clermont-Ferrand Cédex (FR)**

(72) Inventeur : **Chibret, Henri
42, avenue Julien
F-63000 Clermont-Ferrand (FR)**

(74) Mandataire : **Thibon-Littaye, Annick
Cabinet A. THIBON-LITTAYE 11 rue de l'Etang
F-78160 Marly-le-Roi (FR)**

## Description

La présente invention concerne de nouvelles préparations pharmaceutiques présentant une activité anti-allergique quand elles sont administrées localement, plus particulièrement par contact avec la peau et les muqueuses.

Les préparations selon l'invention sont à base d'un principe actif connu. Il s'agit de l'acide N-acétyl-aspartyl-glutamique, dont la préparation est connue et décrite notamment dans le brevet français 2 257 270. L'acide N-acétyl-alpha-aspartyl-glutamique et l'acide N-acétyl-bêta-aspartyl-glutamique peuvent être obtenus et utilisés séparément, mais le plus souvent les préparations selon l'invention contiennent un mélange des formes alpha et bêta. Par ailleurs le principe actif peut être sous la forme de l'acide lui-même ou sous la forme de ses sels biologiquement compatibles connus de l'art antérieur.

Le brevet français cité ci-dessus avait fait connaître les propriétés pharmacologiques de l'acide N-acétyl(alpha et/ou bêta)aspartyl-glutamique dans le traitement de la fatigue cérébrale, des états de chocs et des états asthéniques. Le médicament était alors administré par voie générale, orale ou parentérale.

Toutefois l'activité anti-allergique de l'acide N-acétyl(alpha et/ou bêta)aspartyl-glutamique et de ses sels a également déjà été découverte, ce qui a donné lieu à des utilisations en applications locales. Ainsi le brevet français 2 546 407, qui revendique un médicament à activité anti-allergique pour administration locale, a décrit des compositions destinées à traiter localement des affections telles que les conjonctivites, les rhinites et l'asthme bronchique quand elles ont une origine allergique.

Ce brevet préconise plus particulièrement à cet effet des médicaments qui sont à l'état liquide, sous forme de collyres ou de solutions nasales, qui contiennent l'acide N-acétyl(alpha et/ou bêta)aspartyl-glutamique ou ses sels en solution aqueuse, à une concentration comprise entre 1 et 6 %, et de préférence entre 2 et 4 %, cette proportion étant exprimée en masse d'acide par rapport à la masse totale de la solution.

A l'origine de la présente invention, on a constaté que ces formes liquides à base d'une solution aqueuse d'acide N-acétyl(alpha et/ou bêta)aspartyl-glutamique en solution aqueuse, ont l'inconvénient de présenter une durée d'action limitée, de sorte que des applications fréquemment répétées sont nécessaires pour obtenir l'effet anti-allergique désiré. Ceci conduit à conseiller des posologies qui sont par exemple de deux pulvérisations cinq fois par jour pour une solution nasale, ou six instillations par jour pour un collyre.

On a alors cherché à mettre au point de nouvelles préparations ou compositions pharmaceutiques à base d'acide N-acétyl(alpha et/ou bêta)-aspartyl-glutamique, avec pour objectif d'assurer une meilleure efficacité antiallergique pendant une durée d'action prolongée.

Pour ce faire la présente invention propose une nouvelle composition pharmaceutique pour le traitement local de troubles d'origine allergique, caractérisée en ce qu'elle se présente sous la forme d'un gel ou d'une crème contenant de l'acide N-acétyl(alpha et/ou bêta)aspartyl-glutamique sous forme acide ou salifiée en présence d'un agent viscosifiant et/ou émulsionnant.

Les formes préférées selon l'invention sont celles où l'acide N-acétyl(alpha et/ou bêta)aspartyl-glutamique est salifié, notamment par un métal alcalin ou alcalino-terreux, tel que le sodium, le calcium ou le magnésium. Toutefois on peut également utiliser d'autres sels d'acide N-acétyl(alpha et/ou bêta)aspartyl-glutamique biologiquement compatibles, tels que les sels d'aminoalcool formés avec le dimèthylaminoéthanol ou le diéthylaminoéthanol, ou les sols d'amino-acides basiques tels la lysine.

Les formes préférées selon l'invention sont par ailleurs celles où la composition est constituée sous forme d'un gel au moyen d'un agent viscosifiant en milieu aqueux en proportion appropriée pour conduire à une viscosité comprise entre 20 et 80 000 mPas, et plus particulièrement entre 100 et 30 000 mPas. La concentration en acide N-acétyl(alpha et/ou bêta)aspartyl-glutamique y est avantageusement comprise entre 0,5 et 15 % en poids par rapport à la composition totale, et de préférence entre 1 et 6 %.

Les compositions selon l'invention sont destinées à être utilisées en applications locales, notamment soit en crème ou gel sur la peau, soit en gel pour instillations oculaires ou nasales. Elles montrent alors une activité anti-allergique à effet prolongé, avec un temps de rémanence de trois à six fois supérieur à ce qu'il est actuellement avec les formes commerciales liquides.

On admet ici que l'on désigne par le terme de crème des émulsions qui sont du type eau dans l'huile, alors que le terme de gel est plutôt réservé à des solutions aqueuses gélifiées. Cependant on préférera en général les gels pour le traitement de troubles d'origine allergique du type des conjonctivites et des rhinites et les formes de crèmes pour le traitement des dermatoses allergiques.

L'acide N-acétyl(alpha et/ou bêta)aspartyl-glutamique, sous forme acide ou salifié, peut être préparé selon des méthodes en elles-mêmes connues de l'homme de l'art, telles que celles qui ont été décrites dans le brevet français n° 2 257 270 auquel on se réfèrera en tant que de besoin pour obtenir des composés utiles à la mise en oeuvre de la présente invention. Les formes alpha et bêta sont en général obtenues simultanément en mélange. Les sels préconisés aussi bien que l'acide lui-même sont très solubles dans l'eau à la concentration

2

utile pour la préparation des compositions de l'invention.

L'agent viscosifiant incorporé avec ces composés dans les préparations de l'invention sous forme de gels peut être choisi parmi de nombreux composés en eux-mêmes connus. Parmi ceux-ci on citera plus particulièrement les dérivés de cellulose, notamment ceux de type hydroxyalkyl cellulose, les polymères d'acide acrylique (carbomères), les polysaccharides tels que dextrane, acide hyaluronique, xanthane, les dérivés polyvinyliques tels que l'acrylate de polyvinyle ou la polyvinyl pyrrolidone, ou les protéines comme la gélatine. Des agents viscosifiants appropriés pour la mise en oeuvre de l'invention sont tout spécialement les dérivés de cellulose du type hydroxyalkylcellulose, avec notamment l'hydroxyéthylcellulose, l'hydroxypropylcellulose et l'hydroxypropylméthylcellulose.

Ces composés sont efficaces selon l'invention dans des concentrations comprises de préférence entre 0,2 et 4 % en poids par rapport au poids de la composition totale.

En pratique la nature et la proportion d'agent viscosifiant dans un gel selon l'invention varient non seulement en fonction de la destination d'application de la préparation, mais aussi en fonction de divers constituants additionnels de type en soi connu.

Selon des modes de réalisation préférés de l'invention on aura notamment intérêt à utiliser par exemple :
- dans un gel nasal un viscosifiant cellulosique tel que l'hydroéthylcellulose, en concentration comprise entre 0,25 et 2 % en poids pour obtenir un gel de viscosité comprise entre 30 et 80 000 mPas et plus particulièrement comprise entre 4 000 et 30 000 mPas ;
- dans un gel ophtalmique un viscosifiant cellulosique de type hydroxypropylméthylcellulose, sous une concentration comprise entre 0,25 et 2,5 % en poids, avantageusement de l'ordre de 1,5 à 1,8 % en poids, de manière à obtenir une viscosité du gel comprise entre 20 et 2 000 mPas et plus particulièrement entre 100 et 1000 mPas,
- dans un gel dermique, l'un quelconque des agents viscosifiants indiqués ci-avant, seul ou en mélange avec d'autres, mais de préférence également en proportion comprise entre 0,2 et 4 % pour une proportion de principe actif acide comprise entre 1 et 10 %.

Les autres constituants de telles préparations sous forme de gels peuvent notamment comporter un conservateur anti-microbien capable de garantir la propreté microbienne du produit pendant la période d'utilisation, par exemple de chlorure de benzalkonium, éventuellement additionné de 2-phényléthanol, avec tout autre conservateur dont l'usage est à la portée de l'homme de l'art, par exemple choisi parmi l'acide p-hydroxy benzoïque et ses esters, l'alcool benzylique, le thiomersal, et le chlorobutanol. Les proportions utiles d'un tel conservateur, notamment dans le cas du chlorure de benzalkonium, varient généralement entre 0,005 et 0,025 % en poids de la préparation totale, avec une préférence pour les concentrations comprises entre 0,01 % et 0,02 %.

Parmi d'autres propriétés qui sont importantes pour les préparations de l'invention, mais plus dans le cas d'un gel nasal ou ophtalmique que dans le cas d'un gel dermique, on citera notamment l'acidité et l'osmolalité. L'osmolalité peut être ajustée notamment en incorporant à la composition un agent isotonisant tel que le sorbitol ou un sel ionique tel que les chlorures de sodium, de potassium ou de calcium. La neutralisation acide d'un principe actif à pH basique introduit sous forme d'un sel de l'acide N-acétyl(alpha et/ou bêta)-aspartyl-glutamique s'effectue au moyen d'un acide fort tel que l'acide chlorhydrique, et toute acidité excessive peut être neutralisée par addition d'une base forte telle que la soude.

L'osmolalité d'un gel nasal ou ophtalmique peut être comprise entre 250 et 500 mOsm, celle d'un gel nasal de préférence entre 260 et 340 mOsm, celle d'un gel ophtalmique de préférence entre 280 et 360 mOsmoles. Toutefois, si on utilise l'acide N-acétyl(alpha et/ou bêta)aspartyl-glutamique sous sa forme sel de sodium à une concentration élevée dans les limites de l'invention, elle peut se rapprocher de la valeur de 500 mOsm.

Le pH peut être ajusté à toute valeur avantageusement comprise entre 6,5 et 8,5, avec une préférence pour pH 7,5 pour un gel nasal et pH 7 pour un gel ophtalmique.

Un agent émulsionnant est utilisé au lieu de l'agent viscosifiant quand la préparation selon l'invention est sous forme d'une crème, donc plus particulièrement d'une crème dermique. Son rôle est cependant équivalent dans la mesure où il préserve l'efficacité antiallergique du principe actif et confère à la préparation un effet retard à action prolongée.

Les agents émulsionnants préférés sont ceux qui dérivent d'acides carboxyliques et de polyglycols, tels que les stéarates de polyglycols ou les glycérides de polyoxyéthylène. Leur concentration est alors avantageusement comprise entre 10 et 30 %, et en particulier entre 15 et 25 % en poids de la composition totale. La proportion utile de principe actif, constitué d'acide N-acétyl(alpha et/ou bêta)-aspartyl-glutamique de préférence salifié, est généralement comprise entre 0,5 et 15 % en poids d'acide dans la composition totale, avec cependant une préférence plus nette que dans les cas de gels pour des proportions comprises entre 1 et 6 % de la composition totale.

**EXEMPLE 1 -** Gel nasal

Un gel nasal selon l'invention présente une viscosité comprise entre 30 et 80 000 mPas. Elle pourra avantageusement se situer entre 4000 et 30.000 mPas (millipascal seconde).

La concentration en principe actif peut être comprise entre 1 et 6 %. Elle se situe de préférence entre 2 et 4 %, exprimés en pourcentage d'acide pour 100 g de gel.

L'agent viscosifiant utilisé est une hydroxyéthylcellulose (HEC), vendue sous la dénomination commerciale de NATROSOL 250 HX PHARM. Sa concentration peut varier de 0,25 à 2,0 %.

Le gel contient un conservateur antimicrobien capable de garantir la propreté microbienne pendant la période d'utilisation. Ce conservateur pourra avantageusement être du chlorure de benzalkonium à la concentration de 0,005 à 0,025 % dont on peut renforcer l'action par addition de 2-phényléthanol à une concentration comprise entre 0,2 et 0,6 %. Ce conservateur pourrait être aussi choisi parmi le groupe de l'acide p-hydroxy benzoïque et ses esters, l'alcool benzylique, le thiomersal, le chlorobutanol.

La formulation satisfait à l'essai d'efficacité des agents antimicrobiens (Pharmacopée Française X éd. V 2.1.C, janv. 89).

Du chlorure de sodium est utilisé pour ajuster l'osmolalité à une valeur d'un optimum de 300 mOsm, soit dans les limites de 260 à 340 mOsm, ou plus généralement dans les limites de 250 à 500 mOsm. Il pourrait être remplacé par du chlorure de potassium, du chlorure de calcium ou du sorbitol.

L'acide chlorhydrique et la soude sont utilisés pour ajuster le pH à 7,5, valeur compatible avec l'instillation oculaire. Dans la pratique, le pH peut être compris entre 6,5 et 8,5.

Un tel gel nasal présente par exemple la composition suivante :

| | |
|---|---|
| ‒ Acide N-acétyl-(alpha-bêta)-aspartyl glutamique, sel de magnésium (correspondant à acide NAAGA 4 g) | 6 g |
| ‒ Sorbitol | 0,4 g |
| ‒ Chlorure de benzalkonium | 0,01 g |
| ‒ 2 Phényléthanol | 0,4 g |
| ‒ Acide chlorhydrique 1 N | 0,289 g |
| ‒ Natrosol 250 HX PHARM | 1,8 g |
| ‒ Eau distillée            qsp | 100 g |

La posologie sera avantageusement de 2 à 3 administrations d'une goutte par jour dans chaque narine.

**EXEMPLE 2 :** Préparation du gel nasal selon l'exemple 1

Dissoudre sous agitation dans 45,0 kg d'eau distillée, 5,4 kg d'acide N-acétyl-(alpha-bêta)-aspartyl-glutamique sous forme de son sel de magnésium. Rajouter, tout en maintenant l'agitation, 0,36 kg de SORBITOL puis 0,26 kg d'HCl N, puis une solution (que l'on a fait dissoudre à part) de 0,009 kg de chlorure de benzalkonium dans 1,0 kg d'eau distillée.

Filtrer sur membrane 0,2 microns. Une fois la solution filtrée, rajouter 34,0 kg d'eau distillée et filtrer cette solution sur membrane 0,2 micron.

Dissoudre 0,36 kg de 2-phényléthanol dans la solution obtenue précédemment. Prélever un échantillon et procéder aux contrôles suivants :
- aspect solution : incolore, limpide
- pH = 6,6 - 7,0
- osmolalité : 270 - 330 mOsm.

Disperser sous vide 1,620 kg de Natrosol 250 HX PHARM dans la solution obtenue ci-dessus et agiter pendant 2 heures.

Prélever un échantillon et procéder aux contrôles en cours de fabrication. Si la viscosité est trop faible, ajouter du Natrosol 250 HX PHARM. Transvaser le gel dans des récipients stériles.

La viscosité du gel est mesurée à l'aide d'un viscosimètre BROOKFIELD LVT DV II, comprenant un mobile 34/13 R ou 25/13 R, une chambre SC4/13 R, à la température de 25 °C. La viscosité ainsi mesurée est de 19700 mPas.

Le pH est de 7,3.

L'osmolalité est de 317 mOsm.

**EXEMPLE 3** - Gel ophtalmique

Un gel ophtalmique selon l'invention présente une viscosité comprise entre 20 et 2 000 mPas. Elle pourra avantageusement se situer entre 100 et 1 000 mPas.

La concentration en principe actif peut être comprise entre 1 et 6 %. Elle se situe de préférence entre 2 et 4 %, exprimés en pourcentage d'acide pour 100 g de gel.

L'agent viscosifiant utilisé est une hydroxypropylméthylcellulose (HPMC), vendue sous la dénomination commerciale de METOLOSE 60 SH 4000. Sa concentration peut varier de 0,25 à 2,5 %. Elle sera avantageusement de l'ordre de 1,5 % à 1,8 %.

Le gel est stérilisé par autoclavage pour satisfaire à l'essai de stérilité (Pharmacopée Française X éd. V 2.1.1., juillet 87).

La formulation satisfait à l'essai d'efficacité des agents antimicrobiens (Pharmacopée Française X éd. V 2.1.C, janv. 89).

Du chlorure de sodium est utilisé pour ajuster l'osmolalité à une valeur compatible avec l'instillation dans l'oeil. Il pourrait être remplacé par du chlorure de potassium, du chlorure de calcium ou du sorbitol. L'osmolalité d'un tel gel est voisine de 320 mOsm. Elle peut varier dans les limites de 280 à 360 mOsm, ou plus généralement dans des limites de 250 à 500 mOsm.

L'acide chlorhydrique ou la soude sont utilisés pour ajuster le pH à 7, valeur compatible avec l'instillation oculaire. Dans la pratique, le pH peut être compris entre 6,5 et 7,5.

La préparation complète contient en outre un conservateur comme dans l'exemple 1.

Sa composition est la suivante :

```
- Acide N-acétyl-(alpha-bêta)-aspartyl
  glutamique, sel de sodium, (solution
  aqueuse 50 %)                                            6 g
  (Equivalent à 2,24 g d'acide)
- Hydroxypropylméthylcellulose (HPMC)                    1,5 g
- Chlorure de benzalkonium                              0,01 g
- Chlorure de sodium                                     0,3 g
- Acide chlorhydrique ou NaOH               q.s. pH      7,0
- Eau distillée                             q.s.        100 g
```

La posologie sera avantageusement d'une goutte 1 à 3 fois par jour.

**EXEMPLE 4 :** Préparation d'un gel ophtalmique

Cet exemple concerne la préparation d'un gel répondant à la composition de l'exemple 3.

Dans un bécher de 150 ml, introduire environ 90 ml d'eau désionisée. Ajouter sous agitation magnétique, une quantité d'HPMC double de la concentration centésimale, soit 3 g. Lorsque l'HPMC est totalement dissoute, compléter à 100 g avec de l'eau. Homogénéiser le gel sous agitation magnétique. Stériliser le gel ainsi obtenu à l'autoclave pendant 20 minutes à 120 °C.

Dans un autre bécher, introduire environ 40 ml d'eau désionisée. Ajouter sous agitation magnétique 6 g d'une solution aqueuse à 50 % de sel de sodium de l'acide N-acétyl-(alpha-bêta)-aspartyl-glutamique, 0, 1 g de chlorure de sodium et 1 ml d'une solution de chlorure de benzalkonium à 1 %. Attendre la totale dissolution et filtrer stérilement la solution obtenue (solution A).

Ajouter a cette solution A, 50 g de gel stérilisé. Homogénéiser par agitation magnétique. Ajuster le pH à 7,00 par addition d'une solution de HCl 1N (stérilisée par filtration).

Ajuster à 100 g le gel avec de l'eau stérile. Homogénéiser en final par agitation magnétique.

L'osmolalité de ce gel est de 335 mOsm.

Le pH est de 7,0.

La viscosité, mesurée à l'aide d'un viscosimètre BROOKFIELD LVT DV II comprenant un mobile 18/13 R ou 34/13 R, une chambre SC4/13 R, à la température de 25 °C est de 200 mPas.

**EXEMPLE 5 -** Crème dermique

Une autre forme de réalisation de l'invention consiste en une crème dermique qui, appliquée dans les dermatoses d'origine allergique, présente un effet antipruritique et protège la peau contre les traumatismes persistants tout en lui permettant de cicatriser. Du fait de son absence de toxicité, cette crème est très bien tolérée. Grâce à sa formulation, elle permet à la fois de faire pénétrer le principe actif à travers la peau et d'obtenir un effet antiallergique de longue durée par un maintien du principe actif dans les zones d'application et par sa libération retardée.

Cette forme de réalisation de l'invention est caractérisée en ce qu'elle contient :
- l'acide N-acétyl-(alpha,bêta)-aspartyl-glutamique ou l'un de ses sels à une concentration comprise entre 1 et 6 % ;
- un ou plusieurs émulsionnants choisis pour leur miselbilité à des solvants divers ;
- des conservateurs qui peuvent être choisis parmi le groupe suivant : chlorométhyl-isothiazolone, hydroxybenzoate de méthyle, hydroxybenzoate d'éthyle, acide sorbique ;
- des huiles végétales ou synthétiques qui peuvent être choisies parmi le groupe de l'huile d'onagre, du Cétiol B (dibutyl adipate), de l'huile de paraffine, éventuellement en mélange, en proportion de 5 à 12 % ;
- des facteurs hydratants comme le glycérol, le propylène glycol, en une proportion de 3 à 10 % ;
- des additifs huileux qui contribuent à donner à la préparation une meilleure pénétration cutanée tout en lui donnant une consistance homogène et agréable ;

Les émulsionnants peuvent être notamment choisis parmi le groupe suivant : stéarate de polyéthylèneglycol, Téfose (palmito-stéarate de polyglycols), Labrafil (glycérides de polyoxyéthylène glycolysés). Leur concentration peut être avantageusement de 15 à 25 % en poids par rapport au poids total des constituants de la composition globale.

Un exemple de crème dermique présente la composition ci-après.

| | |
|---|---|
| - Solution aqueuse (50 %) d'acide N-acétyl-(alpha-bêta) aspartyl-glutamique, sel de sodium (équivalent à 3,73 g de NAAGA acide) | 10 g |
| - Tefose 63 (glycérides saturés en $C_{10}$-$C_{18}$ glycolysés et polyoxyétholysés) (Gattefosse) | 16 g |
| - Labrafil M 2130 CS (Palmitostéarate d'éthylène glycol et de polyéthylèneglycol) (Gattefosse) | 2,7 g |
| - Dipélargonate de propylène glycol | 2 g |
| - Huile de paraffine épaisse | 4 g |
| - Cétiol B (dibutyl adipate) | 3 g |
| - Glycérol | 5 g |
| - Hydroxybenzoate de méthyle | 0,1 g |
| - Hydroxybenzoate de propyle | 0,05 g |
| - Eau distillée | q.s. 100 g |

La posologie est de trois applications quotidiennes d'un cylindre de crème d'environ deux centimètres de longueur sur les parties à traiter.

**EXEMPLE 6 -** Etude par radiomarquage au Tc$^{99m}$ de la rémanence d'un gel thérapeutique dans les fosses nasales

La solution et le gel sont marqués par addition de 50 microlitres d'une solution de 37 MBq/ml de diéthylènetriaminopentaacétique, Tc$^{99m}$ à 16 g de solution et de gel.

On suit la rémanence du produit à l'aide d'une gamma caméra grand champ, équipée d'un collimateur basse énergie à trous parallèles et haute sensibilité afin d'améliorer la statistique du comptage. L'examen débute par un enregistrement de 20 minutes, de nouvelles acquisitions sont faites à 30, 45 et 60 minutes pour les deux préparations puis à 90 et 120 minutes pour le gel. Les images numérisées sont stockées sur bandes magnétiques et visualisées sur moniteur couleur ou sorties sur imprimante thermique.

La solution ou le gel sont déposés sous forme d'une goutte de 50 microlitres grâce à l'emploi d'une seringue de chromatographie en phase gazeuse de 100 microlitres, sur la muqueuse du plancher de la fosse nasale au niveau de l'extrémité antérieure du cornet inférieur. Pendant le dépôt, le sujet respire normalement et garde une posture verticale. Il se dispose immédiatement devant la gamma caméra, la face latérale de la narine concernée contre celle-ci. Après l'application, une image latérale de la tête est enregistrée, c'est le point t0, ensuite l'épuration est évaluée en prenant sur une heure, un point toutes les 5 minutes pendant 20 minutes, puis à la trentième et toutes les 15 minutes ensuite.

Chacun des sujets participe à deux essais, l'un avec la solution (S), l'autre avec le gel (G), espacés d'une semaine au moins, dans un ordre défini par le tirage au sort. Les essais ont toujours lieu à la même heure.

Les résultats ont été étudiés quantitativement et qualitativement. Compte tenu de la variabilité inter-sujet, il est apparu que la meilleure façon d'évaluer la rémanence de la préparation était de mesurer, au cours du temps, la décroissance de radioactivité au point de dépôt. L'évolution au cours du temps de l'activité exprimée en pourcentage de la valeur initiale a été établie graphiquement. A partir de cette courbe, il est possible de déterminer le temps pour que 50 % du produit soit éliminé de la zone de dépôt. Ce temps t50 sert de critère pour comparer les deux préparations chez chacun des sujets.

Sur le plan qualitatif, un examen des différentes images successives permet de suivre le trajet et la répartition du produit au cours du temps et donc d'interpréter les données quantitatives.

L'évolution diachronique de l'activité au point de dépôt est répartie graphiquement pour chacun des sujets. Les t50 déterminés à partir des courbes sont exprimés en minutes décimales : le temps nécessaire pour que 50 pour 100 de la préparation soient éliminés de la zone de dépôt varie de 5,8 à 19,8 minutes avec la solution et de 12,6 à 38,2 minutes avec le gel. Le rapport des t50 avec les 2 formes de traitement varie selon les sujets de 1,2 à 4,8. En moyenne il est de 3,1. Ce qui signifie qu'en moyenne le drainage du gel est trois fois plus lent que celui de la solution. Bien entendu, la posologie s'en trouve diminuée dans les mêmes proportions.

**EXEMPLE 7 -** Gel dermique

La préparation suivante est présentée pour application locale sur la peau en vue du traitement des dermatites compte tenu de leur origine souvent allergique. On a choisi d'y introduire le principe actif sous la forme acide, donc non salifié, dans une proportion de 1 à 10 % et de préférence de l'ordre de 4 à 6 %. L'agent viscosifiant utilisé est un carbomère, plus particulièrement un polymère d'acide acrylique du type connu sous la dénomination commerciale CARBOPOL 950 (susceptible de faire l'objet d'une protection par marque). Son acidité est neutralisée au moyen de triéthanolamine.

La composition n'a pas besoin d'ajustement de l'osmolalité. Par contre elle contient en tant que conservateurs, de l'alcool éthylique et de l'ester méthylique de l'acide p-hydroxy benzoïque, ce dernier sous la forme du produit connu sous la dénomination commerciale NIPAGINE (susceptible de faire l'objet d'une protection par marque).

Dans la composition ci-après, comme dans toutes celles qui sont indiquées dans le présent texte, toutes les proportions sont exprimées en poids par rapport au poids global de la composition sauf indications contraires.

| | | |
|---|---|---|
| - NAAGA acide | | 5 g |
| - CARBOPOL ® 950 (Goodrich) | | 0,5 |
| - triéthanolamine | | 1,2 |
| - alcool éthylique | | 10 |
| - NIPAGINE ® | | 0,15 |
| - menthol | qs | |
| - eau désionisée | qsp | 100 g |

Dans une variante de cette composition on a remplacé le mélange d'alcool éthylique et menthol par de l'éthylènediaminetétraacétique (EDTA) utilisé sous forme de son sel sodique. La préparation correspondant répond à la composition ci-après :

| | | |
|---|---|---|
| - NAAGA acide | | 5 g |
| - CARBOPOL ® 950 | | 0,5 |
| - triéthanolamine | | 1,2 |
| - NIPAGINE ® | | 0,15 |
| - EDTA | | 0,1 |
| - eau désionisée | qsp | 100 g |

## Revendications

1. Composition pharmaceutique pour le traitement local de troubles d'origine allergique, caractérisée en ce qu'elle se présente sous la forme d'un gel ou d'une crème contenant de l'acide N-acétyl(alpha et/ou bêta)aspartyl-glutamique sous forme acide ou salifiée en présence d'un agent viscosifiant et/ou émulsionnant.

2. Composition pharmaceutique selon la revendication 1, caractérisée en ce qu'elle contient comme principe actif de l'acide N-acétyl(alpha et/ou bêta) aspartyl-glutamique sous forme d'acide ou de sel biologiquement compatibles, dans une proportion comprise entre 0,5 et 15 % en poids d'acide par rapport au poids total de la composition.

3. Composition selon la revendication 1 ou 2, caractérisée en ce qu'elle se présente sous forme de gel contenant un agent viscosifiant en proportion comprise entre 0,2 et 4 % en poids par rapport au poids de la composition totale.

4. Composition selon la revendication 3, caractérisée en ce que le principe actif est sous forme de sel et en proportion comprise entre 1 et 6 % en poids d'acide par rapport au poids de la composition totale.

5. Composition selon la revendication 3 ou 4, caractérisée en ce que ladite composition sous forme de gel présente une viscosité comprise entre 20 et 80 000 mPas et plus particulièrement entre 100 et 30 000 mPas.

6. Composition selon l'une quelconque des revendications précédentes sous forme de gel nasal ou opthalmique, caractérisée en ce qu'elle comporte comme agent viscosifiant un composé de type hydroxyalkylcellulose.

7. Composition selon la revendication 6, caractérisé en ce que ledit agent viscosifiant est de l'hydroxyéthylcellulose dans un gel nasal destiné au traitement des rhinites d'origine allergique.

8. Composition selon la revendication 7, caractérisée en ce que la concentration d'agents viscosifiant est comprise entre 0,25 et 2 % en poids, et en ce que la viscosité du gel est comprise entre 4 000 et 30 000 mPas.

9. Composition selon la revendication 6, caractérisée en ce que ledit agent viscosifiant est de l'hydroxypropylméthylcellulose dans un gel ophtalmique destiné au traitement des conjonctivites allergiques.

10. Composition selon la revendication 9, caractérisée en ce que la concentration d'agent viscosifiant est comprise entre 0,25 et 2,5 % et de préférence de l'ordre de 1,5 à 1,8 % en poids et en ce que la viscosité du gel est comprise entre 100 et 1 000 mPas.

11. Composition selon l'une quelconque des revendications 1 à 5, sous forme de gel dermique destiné au traitement des dermatites d'origine allergique, caractérisée en ce qu'elle contient de 0,2 à 4 % en poids d'un agent viscosifiant choisi parmi les dérivés de cellulose, notamment de type hydroxyalkylcellulose, les polymères d'acide acrylique, les polysaccharides, les dérivés polyvinyliques ou les protéines comme la gélatine.

12. Composition selon la revendication 1 ou 2, sous forme de crème dermique pour le traitement des dermatites d'origine allergique, caractérisée en ce qu'elle comporte un agent émulsionnant choisi parmi les stéarates de polyglycols ou les glycérides de polyoxyéthylène dans une proportion de 10 à 30 % et de préférence entre 15 et 25 % en poids par rapport au poids de la composition totale.

## Patentansprüche

1. Pharmazeutische Zusammensetzung zur lokalen Behandlung von Erkrankungen allergischer Natur, dadurch gekennzeichnet, daß sie in Form eines Gels oder einer Creme dargestellt wird, die den Inhaltsstoff N-Acetyl-(Alpha und/oder Beta)-Aspartyl-Glutaminsäure in Gestalt der Säure an sich oder ihrer Salze unter Zusatz eines Andickungsmittels und/oder Emulgators enthält.

2. Pharmazeutische Zusammensetzung gemäß Patentanspruch 1, dadurch gekennzeichnet, daß darin der Wirkstoff N-Acetyl-(Alpha und/oder Beta)-Aspartyl-Glutaminsäure in Gestalt der Säure an sich oder biologisch verträglicher Salze in einer Konzentration zwischen 0,5 und 15 % Gewichtsanteil im Verhältnis zum Gesamtgewicht der Zusammensetzung enthalten ist.

3. Zusammensetzung gem. Patentanspruch 1 oder 2, dadurch gekennzeichnet, daß sie in Form eines Gels dargestellt wird, das ein Andickungsmittel in einer Proportion zwischen 0,2 und 4 % Gewichtsanteil im Verhältnis zum Gesamtgewicht der Zusammensetzung enthält.

4. Pharmazeutische Zusammensetzung gemäß Patentanspruch 3, dadurch gekennzeichnet, daß der Wirkstoff in Form von Salzen und in einer Proportion zwischen 1 und 6 % Gewichtsanteil im Verhältnis zum Gesamtgewicht der Zusammensetzung enthalten ist.

5. Zusammensetzung gemäß Patentanspruch 3 oder 4, dadurch gekennzeichnet, daß sie in Form eines Gels eine Viskosität zwischen 20 und 80 000 mPa und insbesondere zwischen 100 und 30 000 mPa aufweist.

6. Zusammensetzung gemäß einem beliebigen der vorstehenden Patentansprüche in Form eines Nasen- oder Augengels, dadurch gekennzeichnet, daß sie als Andickungsmittel eine Zusammensetzung vom Typ Hydroxylalkylzellulose enthält.

7. Zusammensetzung gemäß Patentanspruch 6, dadurch gekennzeichnet, daß das betreffende Andickungsmittel in Form von Hydroxylalkylzellulose in einem Nasengel zur Behandlung von allergisch bedingten Rhinitiden enthalten ist.

8. Zusammensetzung gemäß Patentanspruch 7, dadurch gekennzeichnet, daß die Konzentration der Andickungsmittel zwischen 0,25 und 2 % Gewichtsanteil und die Viskosität des Gels zwischen 4 000 und 30 000 mPa liegt.

9. Zusammensetzung gemäß Patentanspruch 6, dadurch gekennzeichnet, daß das betreffende Andickungsmittel in Form von Hydroxylalkylzellulose in einem Augengel zur Behandlung von allergisch bedingten Bindehautentzündungen enthalten ist.

10. Zusammensetzung gemäß Patentanspruch 9, dadurch gekennzeichnet, daß die Konzentration des Andickungsmittels zwischen 0,25 und 2,5 %, vorzugsweise zwischen 1,5 und 1,8 % Gewichtsanteil und die die Viskosität des Gels zwischen 100 und 1 000 mPa liegt.

11. Zusammensetzung gemäß einem der Patentansprüche 1 - 5, in Form eines Hautgels zur Behandlung allergisch bedingter Dermatosen beschrieben und dadurch gekennzeichnet, daß sie ein Andickungsmittel aus einer Zellulosegruppe, insbesondere vom Typ Hydroxylalkylzellulose, der Akrylsäurepolymerisate, der Polysaccharide, der Polyvinylderivate oder der Proteine, wie die Gelatine, in einer Proportion von 0,2 bis 4 % Gewichtsanteil enthält.

12. Zusammensetzung gemäß Patentanspruch 1 oder 2 in Form einer Hautcreme zur Behandlung allergisch bedingter Dermatosen, dadurch gekennzeichnet, daß sie einen Emulgator aus der Gruppe der Polyglykolstearate oder der Polyoxyäthylenglyzeride in einer Proportion von 10 bis 30 %, vorzugsweise zwischen 15 und 25 % Gewichtsanteil im Verhältnis zum Gesamtgewicht der Zusammensetzung enthält.

## Claims

1. A pharmaceutical composition for the local treatment of disorders of allergic origin, wherein said composition is provided in the form of a gel or a cream containing N-acetyl-(alpha and/or beta)-aspartyl-glutamic acid in an acid or salified form in the presence of a viscosifying and/or emulsifying agent.

2. A pharmaceutical composition according to claim 1, wherein said composition contains as active principle N-acetyl-(alpha and/or beta)-aspartyl-glutamic acid in the form of biologically compatible acid or salt, in a proportion within the range of 0.5 to 15 % by weight of acid with respect to the total weight of the composition.

3. A composition according to claim 1 or 2, wherein said composition is provided in the form of gel containing a viscosifying agent in a proportion within the range of 0.2 to 4 % by weight with respect to the weight of the total composition.

4. A composition according to claim 3, wherein the active principle is in the form of salt and in a proportion within the range of 1 to 6 % by weight of acid with respect to the weight of the total composition.

5. A composition according to claim 3 or 4, wherein said composition in the form of gel has a viscosity within the range of 20 to 80,000 mPas and more particularly 100 to 30,000 mPas.

6. A composition according to any one of preceding claim in the form of nasal or ophthalmic gel, wherein the viscosifying agent contained in said composition is a compound of the hydroxyalkylcellulose type.

7. A composition according to claim 6, wherein said viscosifying agent is hydroxyethylcellulose in a nasal gel intended for the treatment of rhinitis of allergic origin.

8. A composition according to claim 7, wherein the concentration of viscosifying agents is within the range of 0.25 to 2 % by weight, and wherein the viscosity of the gel is within the range of 4000 to 30,000 mPas.

9. A composition according to claim 6, wherein said viscosifying agent is hydroxypropylmethylcellulose in an ophthalmic gel which is intended for the treatment of allergic conjunctivitis.

10. A composition according to claim 9, wherein the concentration of viscosifying agent is within the range of 0.25 to 2.5 % and preferably of the order of 1.5 to 1.8 % by weight and that the viscosity of the gel is within the range of 100 to 1000 mPas.

11. A composition according to any one of claims 1 to 5 in the form of dermal gel intended for the treatment of dermatitis of allergic origin, wherein said composition contains between 0.2 and 4 % by weight of a viscosifying agent selected from the cellulose derivatives, especially of the hydroxyalkylcellulose type, the acrylic acid polymers, the polysaccharides, the polyvinyl derivatives or the proteins such as gelatin.

12. A composition according to claim 1 or 2 in the form of dermal cream for the treatment of dermatitis of allergic origin, wherein said composition contains an emulsifying agent selected from the stearates of polyglycols or the glycerides of polyoxyethylene in a proportion of 10 to 30 % and preferably between 15 and 25 % by weight with respect to the weight of the total composition.